# EUROPÄISCHE PATENTSCHRIFT

(11) **EP 0 490 232 B1**
(45) Veröffentlichungstag und Bekanntmachung des Hinweises auf die Patenterteilung: **23.08.1995**
(21) Anmeldenummer: 91120739.7
(22) Anmeldetag: 03.12.1991
(51) Int. Cl.: G01N 33/28

(54) **Vorrichtung zum Nachweis markierter Mineralöle**
Device for detecting marked mineral oils
Dispositif de détection d'huiles minérales marquées

(30) Priorität: 14.12.1990 DE 4040080
(43) Veröffentlichungstag der Anmeldung: 17.06.1992
(73) Patentinhaber: BASF Aktiengesellschaft, 67063 Ludwigshafen (DE)
(72) Erfinder: Derber, Bernd, W-6703 Limburgerhof (DE); Mauss, Michael, Dr., W-6720 Speyer (DE); Vamvakaris, Christos, Dr., W-6701 Kallstadt (DE)

(56) Entgegenhaltungen:
- EP-A- 0 149 125
- EP-A- 0 311 790
- EP-A- 0 327 163
- US-A- 4 209 302
- US-A- 4 514 503
- US-A- 4 918 020

## Beschreibung

Die Erfindung betrifft eine Vorrichtung zum Nachweis markierter Mineralöle.

Mineralöle, die gegenüber Kraftstoffen mit einer niedrigeren Kaufsteuer belastet sind, beispielsweise Heizöl, werden zur optischen Erkennbarkeit bei Kontrollen der Steuerbehörde gegen Mißbrauch mit einem weiteren Stoff, beispielsweise Furfurol markiert, der bei Reaktion mit einem Nachweisreagenz eine charakteristische Farbe ergibt. In der praktischen Anwendung kann es vorkommen, daß im Mineralöl vorhandene Stoffe die Nachweisreaktion stören. Eine Störung wird dann als solche empfunden, wenn die erwartete charakteristische Farbreaktion nicht eindeutig feststellbar ist. Dies ist insbesondere der Fall, wenn der Markierstoff nicht in der vorgeschriebenen Menge, sondern in geringerer Konzentration vorliegt, zum Beispiel wenn das markierte Mineralöl zum Zweck der Veränderung seiner ursprünglichen Qualität oder seiner steuerlich vorgesehenen Bestimmung manipuliert wurde. Eine solche Manipulation liegt zum Beispiel vor, wenn stuerlich begünstigtes Heizöl mit Dieselkraftstoff vermischt wird und damit Dieselfahrzeuge betrieben werden. Der ursprünglich im Heizöl befindliche Markierstoff ist dann nur noch in geringer Konzentration im Gemisch vorhanden.

Störende Stoffe können natürliche oder synthetische Stoffe sein, die dem Mineralölprodukt zugesetzt oder aufgrund seiner Herkunft vorhanden sind und die Nachweisreaktion beeinträchtigen. Häufig wird bei einer Nachweisreaktion eine zweite, mit dem Heizöl nicht mischbare Phase gebildet. In dieser Phase wird der Markierstoff extrahiert und ergibt mit dem Nachweisreagenz die Farbreaktion, die charakteristisch ist. Wird bei der Durchführung des Nachweises diese charakteristische Farbreaktion nicht beobachtet, kann der Schluß gezogen werden, daß das Mineralöl nicht markiert ist.

Die Störung der Nachweisreaktion kann ferner durch im Mineralöl vorhandene Stoffe hervorgerufen werden, welche von dem Nachweisreagenz in die zweite Phase mitextrahiert werden. Meistens besitzen diese Produkte eine Eigenfarbe oder reagieren selbst in einer Farbreaktion mit dem Nachweisreagenz, wodurch das einwandfreie Identifizieren des Markierstoffes erschwert wird.

Aus der Patentschrift US-A-4 918 020 ist ein Verfahren zur Bestimmung von Kohlenwasserstoffbrennstoffe markierenden Farbstoffen bekannt, wobei der Farbstoff vom Brennstoff mittels einer den Farbstoff adsorbierenden Festphase extrahiert, von der Festphase mit einer Eluierflüssigkeit gelöst und mit einem Nachweisreagenz zur Farbindikation gemischt wird. Für die Durchführung dieses Verfahrens ist ein Analyseablauf in 15 Stufen unter Zuhilfenahme zahlreicher in Analyselabors üblicher Laborgeräte beschrieben. Der damit verbundene hohe Arbeits- und Zeitaufwand sowie die Erfordernis eines umfangreichen Laborplatzes schränkt die Einsatzmöglichkeiten des Verfahrens jedoch ein.

Vorliegender Erfindung liegt daher die Aufgabe zugrunde, eine Vorrichtung zum Nachweis markierter Mineralöle zu entwickeln, mit deren Hilfe Mineralöl markierende Stoffe auch bei verminderter Konzentration im Öl bzw. in dessen Gemisch und bei Vorhandensein störender Stoffe sicher nachgewiesen werden können, wobei die Einsatzmöglichkeiten gegenüber den bekannten Einrichtungen verbessert sein sollen.

Die die Aufgabe lösende Vorrichtung ist gemäß der Erfindung gekennzeichnet durch eine Einrichtung zur Probenahme und Dosierung einer zu untersuchenden Ölprobe, einen daran anschließbaren Behälter für einen den Markierstoff adsorbierenden Stoff, der mit einer Dosiereinrichtung für eine Eluiermittel verbindbar ist, und durch einen unterhalb des Behälterausgangs angeordneten austauschbaren Auffangbehälter für die Ölprobe bzw. das Eluat.

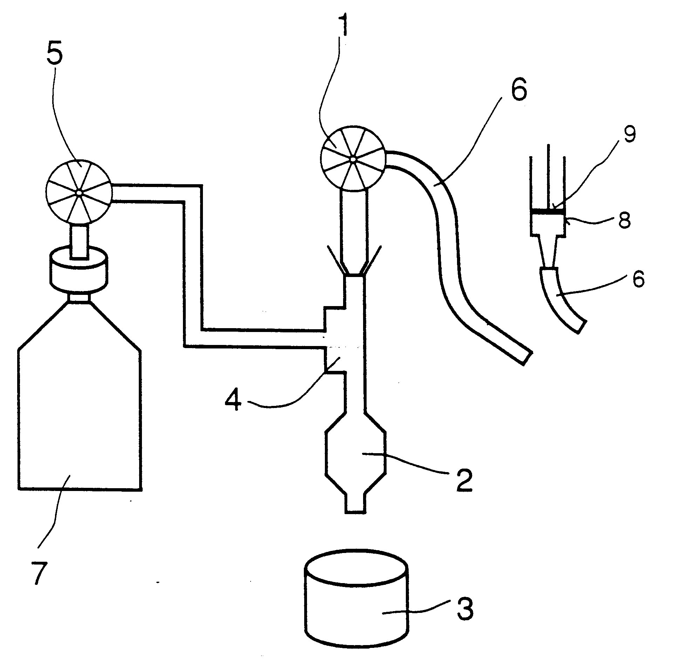

Die Erfindung ist anhand eines in der Zeichnung schematisch dargestellten Ausführungsbeispiel nachfolgend näher beschrieben.

Von dem zu untersuchenden Mineralöl wird aus einem Tank oder Kanister mit Hilfe einer Dosierpumpe 1 über eine Schlauchleitung 6 eine Probe genommen, die anschließend durch einen Behälter 2 geleitet wird, in dem sich ein den Markierstoff des Mineralöls adsorbierender Stoff befindet. Hierfür können verschiedene Produkte eingesetzt werden, beispielsweise in der Analytik für die Säulenchromatographie übliche Produkte. Diese können anorganischer Natur sein, wie zum Beispiel verschiedene Qualitäten von Silicagel oder Kieselgur oder Alumina oder Magnesiumsilikat. Aber auch Produkte organischer Natur kommen in Betracht, wie sie zum Beispiel in der Normal- oder Umkehrphasen-Extraktion verwendet werden.

Nach dem Entfernen des durch den Behälter 2 gelaufenen Mineralöls aus einem unter diesem angeordneten Auffangbehälter 3 oder nach Aufstellen eines neuen Behälters 3 wird der Markierstoff durch Spülen des adsorbierenden Stoffes mit einem geeigneten Eluiermittel selektiv von diesem Stoff gelöst und gelangt dabei im Eluat in den Auffangbehälter 3. Hierzu besteht die Verbindung zwischen der Dosierpumpe 1 und dem Behälter 2 aus einem T-förmigen Verbindungsteil 4, beispielsweise einem Dreiwegehahn, an dessen seitlichem Eingang über eine Dosiereinrichtung 5, beispielsweise eine Kolbenpumpe, ein Vorratsbehälter mit dem Eluiermittel angeschlossen ist.

Im Auffangbehälter 3 kann bereits die für die Nachweisreaktion notwendige Menge an Nachweisreagenz vorhanden sein. Es ist aber ebenso gut möglich, das Nachweisreagenz nachträglich dosiert dem Eluat im Auffangbehälter zuzugeben. Nach Durchmischen des Auffangbehälterinhalts setzt bei Vorhandensein des Markierstoffes die Farbreaktion ein. Eluat und Nachweisreagenz können dabei eine oder zwei Phasen miteinander bilden.

Eine weitere Ausführungsform der erfindungsgemäßen Vorrichtung dann darin bestehen, daß anstelle der Dosierpumpe ein Saugzylinder 8 verwendet wird, mit dem über die Schlauchleitung 6 eine Probe aus einem Tank gezogen werden kann. Nach der Probenahme wird der Saugzylinder auf die Eingangsöffnung des Verbindungsteils 4 gesetzt und die Ölprobe durch Vorschieben des Kolbens 9 im Zylinder durch den Behälter gedrückt.

Die vorstehend beschriebene Vorrichtung erweitert die Einsatzmöglichkeiten des eingangs beschriebenen Nachweisverfahrens, wobei insbesondere ein mobiler Einsatz sowie die Durchführung mit angelerntem Personal möglich sind.

## Patentansprüche

1. Vorrichtung zum Nachweisen markierter Mineralöle, gekennzeichnet durch eine Einrichtung (1) zur Probenahme und Dosierung einer zu untersuchenden Ölprobe, einen daran anschließbaren Behälter (2) für einen den Markierstoff adsorbierenden Stoff, der mit einer Dosiereinrichtung (5) für ein Eluiermittel verbindbar ist, und durch einen unterhalb des Behälterausgangs angeordneten austauschbaren Auffangbehälter (3) für die Ölprobe bzw. das Eluat.

2. Vorrichtung nach Anspruch 1, dadurch gekennzeichnet, daß der Eingang des Behälters (2) mittels eines Dreiwegehahns (4) mit der Einrichtung (1) zur Probenahme und Dosierung der ölprobe und mit der Dosiereinrichtung (5) für das Eluiermittel verbunden ist.

## Claims

1. Apparatus for detecting marked mineral oils, which comprises a means (1) for sampling and metering an oil sample to be investigated, a container (2) for a substance which adsorbs the marking substance, which can be connected to said means and to a metering means (5) for an eluent, and an interchangeable collecting tank (3) for the oil sample or eluate, which tank is arranged underneath the container outlet.

2. Apparatus as claimed in claim 1, wherein the entrance of the container (2) is connected by means of a three-way valve (4) to the means (1) for sampling and metering the oil sample and to the metering means (5) for the eluent.

## Revendications

1. Dispositif pour la détection d'huiles minérales marquées caractérisé par un moyen (1) de prise d'échantillons et de dosage d'un échantillon d'huile à examiner, un récipient (2), raccordable à celui-ci, pour une substance adsorbant la substance de marquage, qui peut être relié à un dispositif de dosage d'éluant (5), et par un récipient collecteur d'échantillon d'huile ou d'éluat (3) remplaçable placé au-dessous de la sortie du récipient (2).

2. Dispositif selon la revendication 1, caractérisé par le fait que l'entrée du récipient (2) est reliée par un robinet à trois voies (4) au moyen (1) de prise et de dosage de l'échantillon d'huile et au dispositif (5) de dosage de l'éluant.
